# EUROPEAN PATENT APPLICATION

(11) **EP 0 999 269 A1**
(43) Date of publication of application: **10.05.2000**
(21) Application number: 98120231.0
(22) Date of filing: 26.10.1998
(51) Int. Cl.: C12N 15/12, C12N 5/10, C07K 14/705, C07K 16/28, A61K 38/17, A01K 67/027, G01N 33/566

(54) **CDNA sequence and genomic organization for a 59.4 kDa neuronal-specific 7 transmembrane protein (pNEU60)**

(71) Applicant: MultiGene Biotech GmbH, 97074 Würzburg (DE)
(72) Inventor: Weber, Bernhard, 97074 Würzburg (DE); Sauer, Christian, 97074 Würzburg (DE)

(57) **Abstract**

The invention pertains to a protein which comprises the same or substantially the same amino acid sequence as that represented by Fig. 3, or a variant of the amino acid sequence having a deletion, addition or substitution of amino acids, an isolated cDNA comprising DNA having a nucleotide sequence encoding this protein, or a DNA hybridizing with this DNA, or a DNA which is synonymous to the said DNAs, a recombinant vector comprising the DNA. The invention furthermore pertains to methods of screening for and detection of pNEU60 mutation carriers with macular degeneration, prenatal pNEU60 screening and diagnosis, gene therapy utilizing recombinant DNA as well as the generation of transgenic animals (knock-in, knock-out, transgenic animals), anti-pNEU60 antibodies and use in screening for modulating drugs.

SEQUENCE LISTING

(1) GENERAL INFORMATION:

(i) APPLICANT:
(A) NAME: MultiGen Biotech GmbH
(B) STREET: Biozentrum, Am Hubland
(C) CITY: Wuerzburg
(D) STATE: -
(E) COUNTRY: Germany
(F) POSTAL CODE (ZIP): 97074
(G) TELEPHONE: 0931-7058-4340
(H) TELEFAX: 0931-7058-4355
(I) TELEX: -

(ii) TITLE OF INVENTION: cDNA sequence and genomic organization
for a
59.4 kDa neuronal-specific 7 transmembrane protein
(pNEU60)

(iii) NUMBER OF SEQUENCES: 19

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 2077 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: exon
(B) LOCATION:1..2077

(xi) SEQUENCE DESCRIPTION: SEQ ID NO: 1:

GGGATGCGCG GAGGCGGTGG CGATGGCGAT GATGCCTCTA GTCCTGCATC
ATCCAGAGCG 60

GCAGGCGGAG CTGGGGTCCG GACTGCGAGA TGGAGGAGGG GCGGCGCTGC
GGCCACCCGG 120

CAGGCTTATC TGTCTTGGGC CTCTTTTGTC ACATATTGCT CATCTGTGAG
CTGAGGCCCT 180

GACTCACTGA GTATTTTTGG GGAGCAGAAG AAGGAGACAT TTCTCTCCGA
AAATGAACTC 240

AACAGGCCAC CTTCAGGATG CCCCCAATGC CACCTCGCTC CATGTGCCTC
ACTCACAGGA 300

AGGAAACAGC ACCTCTCTCC AGGAGGGTCT TCAGGATCTC ATCCACACAG
CCACCTTGGT 360

GACCTGTACT TTTCTACTGG CGGTCATCTT CTGCCTGGGT TCCTATGGCA
ACTTCATTGT 420

CTTCTTGTCC TTCTTCGATC CAGCCTTCAG GAAATTCAGA ACCAACTTTG
ATTTCATGAT 480

CCTGAACCTG TCCTTCTGTG ACCTCTTCAT TTGTGGAGTG ACAGCCCCCA
TGTTCACCTT 540

TGTGTTATTC TTCAGCTCAG CCAGTAGTAT CCCGGATGCT TTCTGCTTCA
CTTTCCATCT 600

CACCAGTTCA GGCTTCATCA TCATGTCTCT GAAGACAGTG GCAGTGATCG
CCCTGCACCG 660

GCTCCGGATG GTGTTGGGGA AACAGCCTAA TCGCACGGCC TCCTTTCCCT
GCACCGTACT 720

CCTCACCCTG CTTCTCTGGG CCACCAGTTT CACCCTTGCC ACCTTGGCTA
CCTTGAAAAC 780

CAGCAAGTCC CACCTCTGTC TTCCCATGTC CAGTCTGATT GCTGGAAAAG
GGAAAGCCAT 840

TTTGTCTCTC TATGTGGTCG ACTTCACCTT CTGTGTTGCT GTGGTCTCTG
TCTCTTACAT 900

CATGATTGCT CAGACCCTGC GGAAGAACGC TCAAGTCAGA AAGTGCCCCC
CTGTAATCAC 960

AGTCGATGCT TCCAGACCAC AGCCTTTCAT GGGGGTCCCT GTGCAGGGAG
GTGGAGATCC 1020

CATCCAGTGT GCCATGCCGG CTCTGTATAG GAACCAGAAT TACAACAAAC
TGCAGCACGT 1080

TCAGACCCGT GGATATACCA AGAGTCCCAA CCAACTGGTC ACCCCTGCAG
CAAGCCGACT 1140

CCAGCTCGTA TCAGCCATCA ACCTCTCCAC TGCCAAGGAT TCCAAAGCCG
TGGTCACCTG 1200

TGTGATCATT GTGCTGTCAG TCCTGGTGTG CTGTCTTCCA CTGGGGATTT
CCTTGGTACA 1260

GGTGGTTCTC TCCAGCAATG GGAGCTTCAT TCTTTACCAG TTTGAATTGT
TTGGATTTAC 1320

TCTTATATTT TTCAAGTCAG GATTAAACCC TTTTATATAT TCTCGGAACA
GTGCAGGGCT     1380

GAGAAGGAAA GTGCTCTGGT GCCTCCAATA CATAGGCCTG GGTTTTTTCT
GCTGCAAACA     1440

AAAGACTCGA CTTCGAGCCA TGGGAAAAGG GAACCTCGAA GTCAACAGAA
ACAAATCCTC     1500

CCATCATGAA ACAAACTCTG CCTACATGTT ATCTCCAAAG CCACAGAAGA
AATTTGTGGA     1560

CCAGGCTTGT GGCCCAAGTC ATTCAAAAGA AAGTATGGTG AGTCCCAAGA
TCTCTGCTGG     1620

ACATCAACAC TGTGGTCAGA GCAGCTCGAC CCCCATCAAC ACTCGGATTG
AACCTTACTA     1680

CAGCATCTAT AACAGCAGCC CTTCCCAGGA GGAGAGCAGC CCATGTAACT
TACAGCCAGT     1740

AAACTCTTTT GGATTTGCCA ATTCATATAT TGCCATGCAT TATCACACCA
CTAATGACTT     1800

AGTGCAGGAA TATGACAGCA CTTCAGCCAA GCAGATTCCA GTCCCCTCCG
TTTAAAGTCA     1860

TGGAGGCTAT AGGATCTTAT GTAAACAGTT TTTGTTTCTG ATAGTAATGG
ACTTTATTCT     1920

AACTTGAGAT CAGTGGCGGA TCAAAACCTA CAAGATTCAA CTGAAAAGTT
GGCAGTTATG     1980

GTTTTCTTTC ATCTGATGTG TCAGTATCTG TTGATTTGCT TTGTAGTTTG
TTGACATCTT     2040

AAGATTTGAT GTGAAAGTTT TAGATTTTTT ACCCTGA
2077

(2) INFORMATION FOR SEQ ID NO: 2:

(i) SEQUENCE CHARACTERISTICS:
(A) LENGTH: 3453 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: exon
(B) LOCATION:1..3453

(xi) SEQUENCE DESCRIPTION: SEQ ID NO: 2:

AACTGGAAGC CGCCGCCAAT TTAATTCATG AGGAAAAATA TTAGCCGCAG
CCTAAGTGAG 60

GAGCTAGTTA GGACGTCTTA AAAGCGCGGG GAACGAGGAG GAGGTGGGCT
GCGGGCAGGG 120

TAAGCAGCCT AGGGAGGCCC GCAAAGACCT CCCTGGGTCC CTGCGTGCCA
TCAGTACCCC 180

TATCTTTCCC TTCCTTCCTT ACCACTCCAC CTTCGTCTGC GTTTCAGGTT
TTGGCTATTT 240

CAGGGAGCTT GAGTTCCCCA AACAGCAAAA CTGCGAGCCT GAAAAACCTT
CTGCTCAAGC 300

GCCCCTCGTG AGGCGGGCAG CGGGAGTGGC CGGCTCTGGG CCCCAGGAGG
CAGGCGACGG 360

AGGCGCGGAG GGTGCGGGGC GCAGCGGGAC GCCAGGCCCG GGTGTCCGGC
GGAGGGGCGG 420

TGCCCTCGGC GTCTCCGTGA CTCGCCTCTG CGCCGCGTCT TGCCGCGGCT
CCCGGGATGC 480

GCGGAGGCGG TGGCGATGGC GATGATGCCT CTAGTCCTGC ATCATCCAGA
GCGGCAGGCG 540

GAGCTGGGGT CCGGACTGCG AGATGGAGGA GGGGCGGCGC TGCGGCCACC
CGGCAGGTGA 600

GAGCCGCGGG CCCCTGGAGG AGGACAACCC CACGATGCCG GAGACGGCTC
CCGGAGGCTG 660

GCGGGATAGC GAGGACGGCG GCTGCGCTGG GCCAGGCCCG GCTCCGCGTA
CCTGTCTTCC 720

TGGTGCGCCT GCAGGGTAGC CTTCTCGCCC GTCCCTGCCG GTTCCCAGGC
TCTGCTCGGC 780

CGTGGAACCC CACCCACCCA CCCACCGCCC CTACCCTGGC TGAGCCCTCC
TAACCCACCA 840

CCCCTCTGCG GCATTCTTTT GCAAGCTTAC CTGGCCCGGC CTAGGCCTCC
TTACCGTCAC 900

CTCACCCTTC TCCGGGAAGC CCTACCCACC GCCAGCCCTC ACGGGGGTCG
ACCTCCCTGC 960

CCGAGTTCCA TCTGTGTCTA GCTGGGGTCC TGGCTCTTCA TCCCTCCCAT
CAAATGACTC 1020

AGGTCTTCCA TCCATTAGCT GCTCCGGGTT CAATGTAGTT CTACTGGATA
GAAAGAGCAG 1080

GGCTTTGAAA TCAGATAATT GACCTTGGGA AGTTATCTAA CCTCCCTGAG
GTTTTCCTGA 1140

TCGATAGAAA TACAATGGGA ATCACAATAA TACAGAACCT TGCTCTCTGT
GAGGATTAGG 1200

CTTTGAAAAT GCTAACCTAA GACATTTTGG GGCGCGCGGG GAATGGGGTT
GGAAGAATTA 1260

GAGAGAGAGA GAGCTGGTTT GGTGGTGCAT GCTGTAGTCC AGCTACTGAG
CTAGCAGAGA 1320

CTGCTGAGCA CAGAGTCAGT CAGCTGGCAC ATGTAAGACC CATGCTATAA
TGATAATTTT 1380

TCTCTCAAGT GAGAGATACT AGTACTAATA CTACTACTAA TAATGAGATC
ACTGTATGAA 1440

TTCCTTTCTG TTTCTACATT AATCCCTTTC TGTTTCTGGG TGTGCATTTT
TGTGTGTAGG 1500

CTTATCTGTC TTGGGCCTCT TTTGTCACAT ATTGCTCATC TGTGAGCTGA
GGCCCTGACT 1560

CACTGAGTAT TTTTGGGGAG CAGAAGAAGG AGACATTTCT CTCCGAAAAT
GAACTCAACA 1620

GGCCACCTTC AGGATGCCCC CAATGCCACC TCGCTCCATG TGCCTCACTC
ACAGGAAGGA 1680

AACAGCACCT CTCTCCAGGA GGGTCTTCAG GATCTCATCC ACACAGCCAC
CTTGGTGACC 1740

TGTACTTTTC TACTGGCGGT CATCTTCTGC CTGGGTTCCT ATGGCAACTT
CATTGTCTTC 1800

TTGTCCTTCT TCGATCCAGC CTTCAGGAAA TTCAGAACCA ACTTTGATTT
CATGATCCTG 1860

AACCTGTCCT TCTGTGACCT CTTCATTTGT GGAGTGACAG CCCCCATGTT
CACCTTTGTG 1920

TTATTCTTCA GCTCAGCCAG TAGTATCCCG GATGCTTTCT GCTTCACTTT
CCATCTCACC 1980

AGTTCAGGCT TCATCATCAT GTCTCTGAAG ACAGTGGCAG TGATCGCCCT
GCACCGGCTC 2040

CGGATGGTGT TGGGGAAACA GCCTAATCGC ACGGCCTCCT TTCCCTGCAC
CGTACTCCTC 2100

ACCCTGCTTC TCTGGGCCAC CAGTTTCACC CTTGCCACCT TGGCTACCTT
GAAAACCAGC 2160

AAGTCCCACC TCTGTCTTCC CATGTCCAGT CTGATTGCTG GAAAAGGGAA
AGCCATTTTG 2220

TCTCTCTATG TGGTCGACTT CACCTTCTGT GTTGCTGTGG TCTCTGTCTC
TTACATCATG 2280

ATTGCTCAGA CCCTGCGGAA GAACGCTCAA GTCAGAAAGT GCCCCCCTGT
AATCACAGTC 2340

GATGCTTCCA GACCACAGCC TTTCATGGGG GTCCCTGTGC AGGGAGGTGG
AGATCCCATC 2400

CAGTGTGCCA TGCCGGCTCT GTATAGGAAC CAGAATTACA ACAAACTGCA
GCACGTTCAG 2460

ACCCGTGGAT ATACCAAGAG TCCCAACCAA CTGGTCACCC CTGCAGCAAG
CCGACTCCAG 2520

CTCGTATCAG CCATCAACCT CTCCACTGCC AAGGATTCCA AAGCCGTGGT
CACCTGTGTG 2580

ATCATTGTGC TGTCAGTCCT GGTGTGCTGT CTTCCACTGG GGATTTCCTT
GGTACAGGTG 2640

GTTCTCTCCA GCAATGGGAG CTTCATTCTT TACCAGTTTG AATTGTTTGG
ATTTACTCTT 2700

ATATTTTTCA AGTCAGGATT AAACCCTTTT ATATATTCTC GGAACAGTGC
AGGGCTGAGA 2760

AGGAAAGTGC TCTGGTGCCT CCAATACATA GGCCTGGGTT TTTTCTGCTG
CAAACAAAAG 2820

ACTCGACTTC GAGCCATGGG AAAAGGGAAC CTCGAAGTCA ACAGAAACAA
ATCCTCCCAT 2880

CATGAAACAA ACTCTGCCTA CATGTTATCT CCAAAGCCAC AGAAGAAATT
TGTGGACCAG 2940

GCTTGTGGCC CAAGTCATTC AAAAGAAAGT ATGGTGAGTC CCAAGATCTC
TGCTGGACAT 3000

CAACACTGTG GTCAGAGCAG CTCGACCCCC ATCAACACTC GGATTGAACC
TTACTACAGC 3060

ATCTATAACA GCAGCCCTTC CCAGGAGGAG AGCAGCCCAT GTAACTTACA
GCCAGTAAAC     3120

TCTTTTGGAT TTGCCAATTC ATATATTGCC ATGCATTATC ACACCACTAA
TGACTTAGTG     3180

CAGGAATATG ACAGCACTTC AGCCAAGCAG ATTCCAGTCC CCTCCGTTTA
AAGTCATGGA     3240

GGCTATAGGA TCTTATGTAA ACAGTTTTTG TTTCTGATAG TAATGGACTT
TATTCTAACT     3300

TGAGATCAGT GGCGGATCAA AACCTACAAG ATTCAACTGA AAAGTTGGCA
GTTATGGTTT     3360

TCTTTCATCT GATGTGTCAG TATCTGTTGA TTTGCTTTGT AGTTTGTTGA
CATCTTAAGA     3420

TTTGATGTGA AAGTTTTAGA TTTTTTACCC TGA
3453

(2) INFORMATION FOR SEQ ID NO: 3:

(i) SEQUENCE CHARACTERISTICS:
(A) LENGTH: 540 amino acids
(B) TYPE: amino acid
(C) STRANDEDNESS: single
(D) TOPOLOGY: linear

(ii) MOLECULE TYPE: peptide

(ix) FEATURE:
(A) NAME/KEY: Peptide
(B) LOCATION:1..540

(xi) SEQUENCE DESCRIPTION: SEQ ID NO: 3:

Met Asn Ser Thr Gly His Leu Gln Asp Ala Pro Asn Ala Thr Ser Leu
1               5                   10                  15

His Val Pro His Ser Gln Glu Gly Asn Ser Thr Ser Leu Gln Glu Gly
            20                  25                  30

Leu Gln Asp Leu Ile His Thr Ala Thr Leu Val Thr Cys Thr Phe Leu
        35                  40                  45

Leu Ala Val Ile Phe Cys Leu Gly Ser Tyr Gly Asn Phe Ile Val Phe
    50                  55                  60

Leu Ser Phe Phe Asp Pro Ala Phe Arg Lys Phe Arg Thr Asn Phe Asp
65                  70                  75                  80

Phe Met Ile Leu Asn Leu Ser Phe Cys Asp Leu Phe Ile Cys Gly Val
                85                  90                  95

Thr Ala Pro Met Phe Thr Phe Val Leu Phe Phe Ser Ser Ala Ser Ser
            100                 105                 110

Ile Pro Asp Ala Phe Cys Phe Thr Phe His Leu Thr Ser Ser Gly Phe
        115                 120                 125

Ile Ile Met Ser Leu Lys Thr Val Ala Val Ile Ala Leu His Arg Leu
    130                 135                 140

Arg Met Val Leu Gly Lys Gln Pro Asn Arg Thr Ala Ser Phe Pro Cys
145                 150                 155                 160

Thr Val Leu Leu Thr Leu Leu Leu Trp Ala Thr Ser Phe Thr Leu Ala
                165                 170                 175

Thr Leu Ala Thr Leu Lys Thr Ser Lys Ser His Leu Cys Leu Pro Met
            180                 185                 190

Ser Ser Leu Ile Ala Gly Lys Gly Lys Ala Ile Leu Ser Leu Tyr Val
        195                 200                 205

Val Asp Phe Thr Phe Cys Val Ala Val Val Ser Val Ser Tyr Ile Met
    210                 215                 220

Ile Ala Gln Thr Leu Arg Lys Asn Ala Gln Val Arg Lys Cys Pro Pro
225                 230                 235                 240

Val Ile Thr Val Asp Ala Ser Arg Pro Gln Pro Phe Met Gly Val Pro
                245                 250                 255

Val Gln Gly Gly Gly Asp Pro Ile Gln Cys Ala Met Pro Ala Leu Tyr
            260                 265                 270

Arg Asn Gln Asn Tyr Asn Lys Leu Gln His Val Gln Thr Arg Gly Tyr
        275                 280                 285

Thr Lys Ser Pro Asn Gln Leu Val Thr Pro Ala Ala Ser Arg Leu Gln
    290                 295                 300

Leu Val Ser Ala Ile Asn Leu Ser Thr Ala Lys Asp Ser Lys Ala Val
305                 310                 315                 320

Val Thr Cys Val Ile Ile Val Leu Ser Val Leu Val Cys Cys Leu Pro
                325                 330                 335

Leu Gly Ile Ser Leu Val Gln Val Val Leu Ser Ser Asn Gly Ser Phe
            340                 345                 350

Ile Leu Tyr Gln Phe Glu Leu Phe Gly Phe Thr Leu Ile Phe Phe Lys
        355                 360                 365

Ser Gly Leu Asn Pro Phe Ile Tyr Ser Arg Asn Ser Ala Gly Leu Arg
    370                 375                 380

Arg Lys Val Leu Trp Cys Leu Gln Tyr Ile Gly Leu Gly Phe Phe Cys
385                 390                 395                 400

Cys Lys Gln Lys Thr Arg Leu Arg Ala Met Gly Lys Gly Asn Leu Glu
                405                 410                 415

Val Asn Arg Asn Lys Ser Ser His His Glu Thr Asn Ser Ala Tyr Met
            420                 425                 430

Leu Ser Pro Lys Pro Gln Lys Lys Phe Val Asp Gln Ala Cys Gly Pro
        435                 440                 445

Ser His Ser Lys Glu Ser Met Val Ser Pro Lys Ile Ser Ala Gly His
    450                 455                 460

Gln His Cys Gly Gln Ser Ser Ser Thr Pro Ile Asn Thr Arg Ile Glu
465                 470                 475                 480

Pro Tyr Tyr Ser Ile Tyr Asn Ser Ser Pro Ser Gln Glu Glu Ser Ser
                485                 490                 495

Pro Cys Asn Leu Gln Pro Val Asn Ser Phe Gly Phe Ala Asn Ser Tyr
            500                 505                 510

Ile Ala Met His Tyr His Thr Thr Asn Asp Leu Val Gln Glu Tyr Asp
        515                 520                 525

Ser Thr Ser Ala Lys Gln Ile Pro Val Pro Ser Val
    530                 535                 540

(2) INFORMATION FOR SEQ ID NO: 4:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..18

(xi) SEQUENCE DESCRIPTION: SEQ ID NO: 4:

GCATCATCCA GAGCGGCA
18

(2) INFORMATION FOR SEQ ID NO: 5:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 5:

GAAAGAAAAC CATAACTGCC
20

(2) INFORMATION FOR SEQ ID NO: 6:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(ix) FEATURE:

(A) NAME/KEY: exon
(B) LOCATION:1..19

(xi) SEQUENCE DESCRIPTION: SEQ ID NO: 6:

ATCTCATCCA CACAGCCAC
19

(2) INFORMATION FOR SEQ ID NO: 7:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 7:

TAATCCCTTT CTGTTTCTGG
20

(2) INFORMATION FOR SEQ ID NO: 8:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 8:

CCAGTTCAGG CTTCATCATC
20

(2) INFORMATION FOR SEQ ID NO: 9:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..19

(xi) SEQUENCE DESCRIPTION: SEQ ID NO: 9:

TGTGCAGGGA GGTGGAGAT
19

(2) INFORMATION FOR SEQ ID NO: 10:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 10:

GTCCTGGTGT GCTGTCTTCC
20

(2) INFORMATION FOR SEQ ID NO: 11:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:

(A) NAME/KEY: exon
(B) LOCATION:1..20

(xi) SEQUENCE DESCRIPTION: SEQ ID NO: 11:

CAGAAACAAA TCCTCCCATC
20

(2) INFORMATION FOR SEQ ID NO: 12:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..18

(xi) SEQUENCE DESCRIPTION: SEQ ID NO: 12:

AACTCTTTTG GATTTGCC
18

(2) INFORMATION FOR SEQ ID NO: 13:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 13:

AGGACAAGAA GACAATGAAG
20

(2) INFORMATION FOR SEQ ID NO: 14:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..14

(xi) SEQUENCE DESCRIPTION: SEQ ID NO: 14:

CGATCACTGC CACTGTCTTC
20

(2) INFORMATION FOR SEQ ID NO: 15:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..19

(xi) SEQUENCE DESCRIPTION: SEQ ID NO: 15:

CCGGCATGGC ACACTGGAT
19

(2) INFORMATION FOR SEQ ID NO: 16:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:

(A) NAME/KEY: exon
(B) LOCATION:1..20

(xi) SEQUENCE DESCRIPTION: SEQ ID NO: 16:

AAACTGGTAA AGAATGAAGC
20

(2) INFORMATION FOR SEQ ID NO: 17:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 17:

CTGTGGCTTT GGAGATAACA
20

(2) INFORMATION FOR SEQ ID NO: 18:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 18:

CTGTGGCTTT GGAGATAACA
20

(2) INFORMATION FOR SEQ ID NO: 19:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 19:

GAAAGAAAAC CATAACTGCC
20

## Description

### Field of the invention

The present invention relates to the cloning and sequencing of the human cDNA molecule and the genomic structure of a neuronal-specific gene encoding a putative 59.4 kDa 7 transmembrane protein (pNEU60). The present invention also relates to methods of screening for and detection of pNEU60 mutation carriers with macular degeneration, prenatal pNEU60 screening and diagnosis, gene therapy utilizing recombinant DNA as well as the generation of transgenic animals (knock-in, knock-out, transgenic animals), anti-pNEU60 antibodies and use in screening for modulating drugs.

### Background of the Invention

First described in 1855 (Donders 1855), age-related macular degeneration (AMD) is now recognized as the most common cause of visual morbidity in the developed world (Paetkau et al. 1978, Leibowitz et al. 1980, Banks and Hutton 1981, Ghafour et al. 1983, Hyman 1987, Grey et al. 1989, Yap and Weatherill 1989, Heiba et al. 1994). The prevalence of AMD in persons over 52 was found to be 9% increasing to more than 25% in persons over the age of 75 (Hyman et al. 1983). Projected estimates indicate that by the year 2020 as many as 7.5 million individuals over 65 years may suffer from central vision loss due to AMD (Pizzarello 1987). As the number of older people in industrialized countries increases, the associated social and economic consequences of AMD are destined to increase in the next millenium unless preventive or therapeutic treatments can be found.

Histologically, an increasing accumulation of yellowish lipofuscin-like particles within the retinal pigment epithelium (RPE) can be observed with age (Feeney 1978). They likely represent an early stage in the evolution of AMD pathology and are followed by secondary complications frequently associated with loss of visual acuity (Pauleikhoff et al. 1990 and 1992). It is thought that the lipofuscin-like deposits represent remnants of undigested phagocytosed photoreceptor outer segment membranes which, in the normal process, are excreted basally through Bruch's membrane into the choriocapillaris. Over time, incomplete digestion and transport of lipofuscin-like particles affect Bruch's membrane and lead to its progressive destruction seen by electron microscopy as an abnormal thickening of the inner collagenous layer of the membrane (Hogan and Alvarado 1967, Sarks 1976, Feeney-Burns and Ellersieck 1985, Pauleikhoff et al. 1990). The deposits in the RPE and Bruch's membrane consist largely of lipids although their exact composition varies widely between individuals with some depositing more polar phospholipids while in others predominantly apolar neutral lipids can be found (Holz et al. 1994, Pauleikhoff et al. 1990).

These individual differences in composition are thought to be the basis for the clinical heterogeneity in AMD (Green et al. 1985). While some patients present with an ingrowth of vessels from the choriocapillaris through Bruch's membrane (neovascularization) (Bressler et al. 1982), others show pigment epithelial detachment due to excudation underneath the RPE (Gass 1967, Green et al. 1985), and a third group of patients experiences a slow decrease of visual loss due to atrophic changes in the RPE and the overlying sensory neuroretina (Maguire and Vine 1986). Although much less common (∼10% of all AMD patients) the excudative/neovascular form of AMD accounts for more than 80% of blindness with a visual acuity of ≤ 20/200 (Bressler et al. 1988).

AMD is a complex disease caused by exogenous as well as endogenous factors (Meyers and Zachary 1988; Seddon et al. 1997). In addition to environmental factors, several personal risk factors such as hypermetropia, light skin and iris colour, elevated serum cholesterol levels, hypertension or cigarette smoking have been suggested (Hyman et al. 1983, Klein et al. 1993, Sperduto and Hiller 1986, The Eye Disease Case-Control Study Group 1992, Bressler and Bressler 1995). A genetic component for AMD has been documented by several groups (Gass 1973, Piguet et al. 1993, Silvestri et al. 1994) and has lead to the hypothesis that the disease may be triggered by environmental/individual factors in those persons who are genetically predisposed. The number of genes which, when mutated, can confer susceptibility to AMD is not known but may be numerous. The photoreceptor-specific ATP-binding cassette (ABCR) gene may represent the first example of a gene predisposing to AMD (Allikmets et al. 1997) although methodological problems in study design and interpretation of data have given rise to great controversy (Dryja et al. 1998, Klaver et al. 1998).

Extensive research is currently in progress and is directed towards the identification of genes conferring susceptibility to AMD. However, the late onset of symptoms generally in the 7th decade of life as well as the clinical and likely genetic heterogeneity make it difficult, if not impossible, to apply conventional approaches for the identification of genes predisposing to AMD. We therefore have designed a strategy which aims at the identification of genes that are expressed exclusively or predominantly in the human retina and that makes use of the extensive information available at the public expressed sequence tag databases (dbEST). We argue that genes that are expressed exclusively or predominantly in the human retina should play an important functional role in this tissue and therefore may causally be involved in the etiology of AMD.

It is the object of the present invention to provide a human cDNA molecule and the genomic exon/intron organization of a novel gene, tentatively termed pNEU60, which is expressed exclusively in neuronal tissue with a major site of expression in the sensory neuroretina. Preliminary mutational analyses in AMD patients have revealed genetic alterations in this gene which are thought to be associated with disease pathology. The cloning and sequencing of pNEU60 should facilitate new and improved methods of diagnosis and prophylactic/therapeutic treatments of AMD.

### Summary of the invention

The foregoing object has been achieved by providing an isolated human cDNA molecule (pNEU60) as well as its genomic exon/intron organization. Preliminary mutational analyses in 200 patients with AMD have resulted in the identification of several mutations in this gene strongly suggesting disease association. Investigations to clarify the exact nature of the molecular pathology of these mutations are presently in progress. The invention encompasses this pNEU60 cDNA molecule, the nucleotide sequence of this cDNA, the nucleotide sequence of its genomic exon/intron structure and the putative amino acid sequence of the pNEU60 protein.

Also comprehended by this invention are oligonucleotide primers comprising the full length cDNA molecule or its complementary strand as well as forward and reverse oligonucleotide primers designed from intervening and exonic sequences of the pNEU60 gene that allow the amplification of isolated exonic sequences by the polymerase chain reaction. Such primers are particularly useful and will provide physicians with an enhanced ability to diagnose patients with macular degeneration. The present invention also relates to methods of screening for and detection of pNEU60 mutation carriers including prenatal pNEU60 screening and diagnosis.

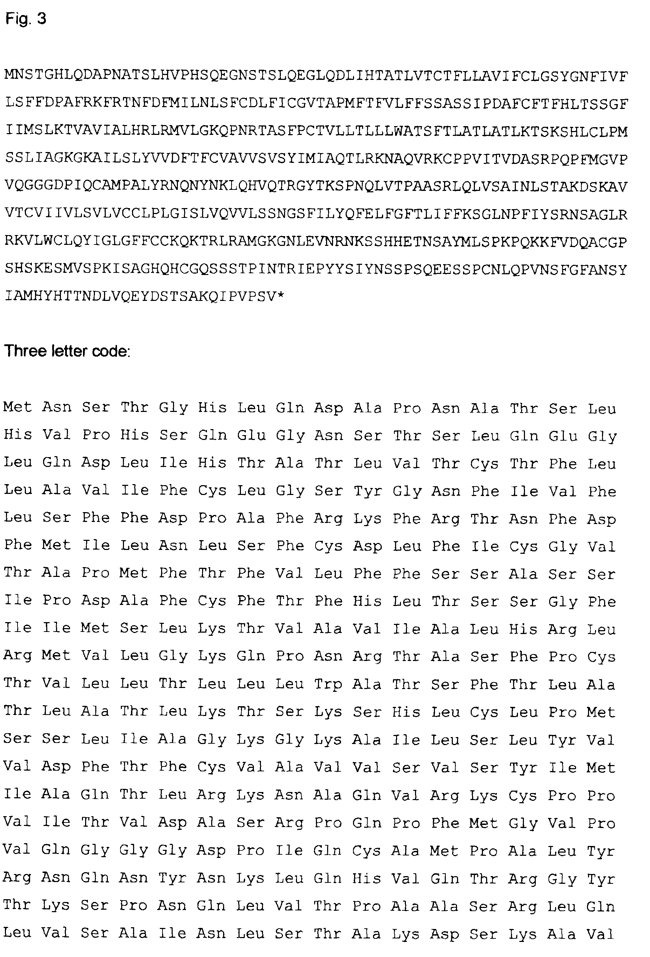

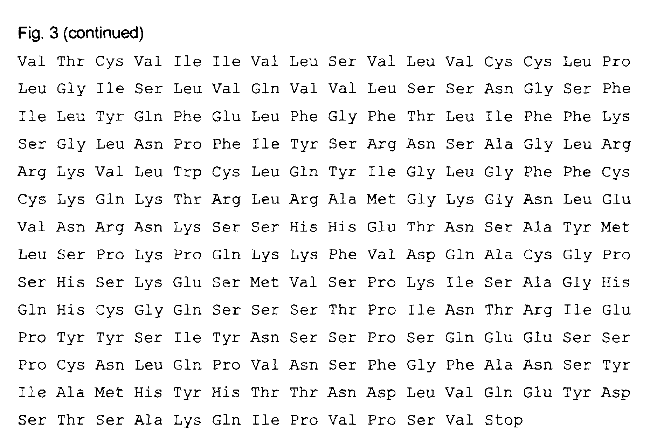

Having provided the isolated human pNEU60 cDNA sequence, also comprehended by this invention is the genomic gene from which the pNEU60 cDNA is derived. The present invention also provides for the use of the pNEU60 cDNA, the corresponding genomic gene and derivatives thereof, and of the pNEU60 protein, and derivatives thereof in aspects of diagnosis and treatment of macular degeneration. Finally, the invention pertains to a protein which comprises the same or substantially the same amino acid sequence (at least 200 amino adds) as that represented by Fig. 3 and SEQ ID NO. 3, or a variant of the amino acid sequence having a deletion, addition or substitution of 1 to 10 amino acids, or its salt.

Another aspect of the invention is the use of the pNEU60 protein as a target for drug and gene therapy in the treatment of macular degeneration pathology. This includes the generation and utilization of pNEU60-targeted animal models (knock-in, knock-out, transgenic animals) and anti-pNEU60 antibodies that specifically detect the pNEU60 protein.

The foregoing and other features and advantages of the invention will become more apparent from the following detailed description and accompanying drawings.

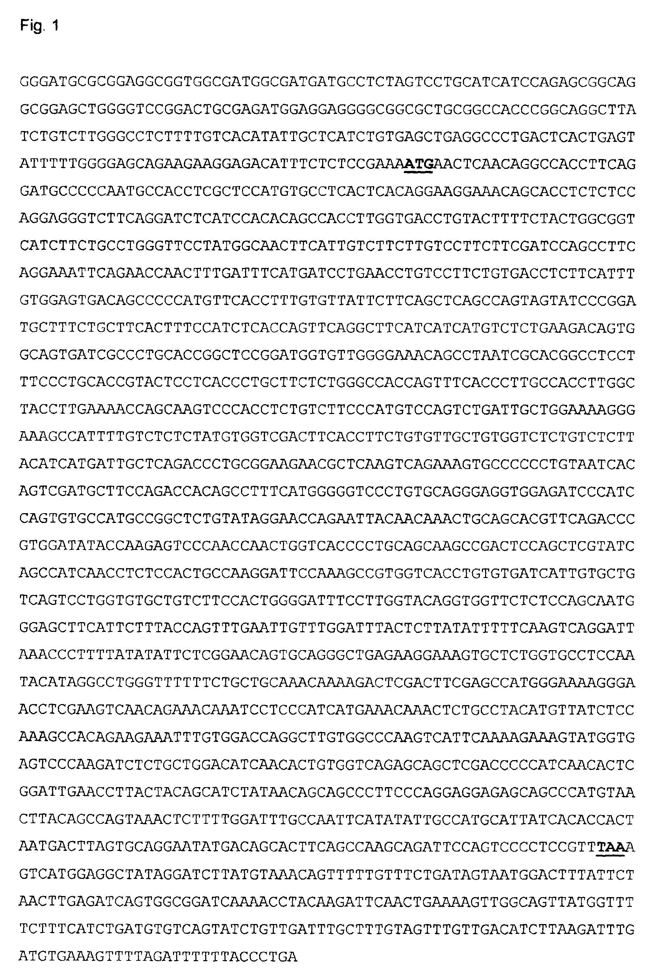

One aspect of the invention is an isolated cDNA comprising
a) DNA having a nucleotide sequence encoding the said protein,
b) a DNA hybridizing with the DNA of a), or
   a DNA which is synonymous to a) or b) due to the degeneracy of the genetic code, especially the isolated DNA sequence according having
   a) the nucleotide sequence represented by Fig. 1 and SEQ ID NO. 1,
   b) DNA hybridizing with the DNA of a), or
      a DNA which is synonymous to a) or b) due to the degeneracy of the genetic code.

A DNA comprising a nucleotide sequence with at least a 65 % homology with the nucleotide sequence is also within the scope of the invention.

Furthermore within the scope of the invention are:

A recombinant vector comprising the disclosed DNA molecules.

Transgenic host cells such as COS7, fibroblast cell lines or retinal pigment epithelial cell lines.

A process for preparing the protein which comprises cultivating the transformant to form the protein.

A method of screening for modulators in well known assays using constructs such as pNEU60 promoter luciferase or green fluorescent protein hybrids or screening for interacting proteins or factors using state of the art technologies like the interaction trap technology to screen for interacting substances of pNEU60 or isolated domains of pNEU60.

A method of screening chemical libraries comprising transformed cell lines

A compound which alters / reacts with at least one epitope of the protein and which is obtained by screening methods utilizing the pNEU60 cDNA or protein molecule.

Use of antibodies against the pNEU60 protein for diagnostic or therapeutic purposes.

A pharmaceutical composition comprising as an effective component the protein or a partial peptide of it, and a pharmaceutically acceptable carrier or diluent.

### Detailed Description of the Invention

### Materials and Methods

### Isolation of pNEU60 cDNA

To identify expressed sequence tag (EST) clones exclusively or predominantly expressed in the human retina the publically available EST databases at the National Center for Biotechnology Information (NCBI) at the National Institutes of Health (NIH), Bethesda, Maryland (http://www2.ncbi.nlm.nih.gov/cgi-bin/genbank/) and at the Institute for Genomic Research in Rockville, MD (http://www.tigr.org/tdb/tdb.html) were searched for multiple hits in retinal EST sequences (≥ 3). The stringency was set to 80% requiring that at least 8 out of 10 hits in the databases must include retina-derived EST clones. Based on these criteria, we identified a three overlapping retina-derived EST clones (ys91a03, ze59a07, ze65c05). Approximatley 300 base pairs of high-quality sequences were available from the 5'- and 3'-ends of these clones, respectively. To isolate the corresponding cDNA we designed PCR primers from the 5'-(DHRD.91.r12; 5'-GCA TCA TCC AGA GCG GCA-3') and 3'-sequences (DHRD.91.s11; 5'-GAA AGA AAA CCA TAA CTG CC-3') amplifying a 1.945 bp transcript in reverse transcribed human retinal RNA. The cDNA fragment was completely sequenced using walking primer technology and thermo sequenase radiolabeled terminator cycle sequencing (Amersham, Life Science).

### 5'-RACE

To isolate the complete 5'-end of the cDNA the technique of 5'-RACE (rapid amplification of cDNA ends) was used. 5'-RACE is a procedure for amplification of nucleic acid sequences from a messenger RNA template between a defined internal site and unknown sequences at the 5'-end of the mRNA (Frohman et al. 1988). First strand cDNA synthesis was primed using the gene-specific antisense oligonucleotide DHRD.91 .s8 (5'-CGA TCA CTG CCA CTG TCT TC-3'). Following cDNA synthesis, the first strand product is purified from unincorporated dNTPs and remaining primers DHRD.91.s8. A homopolymeric tail is then added to the 3'-end of the cDNA using terminal deoxynucleotidyl transferase (TdT) and dCTP. PCR amplification is accomplished using Taq DNA polymerase, the nested gene-specific primer DHRD.91.s4 (5'-AGG ACA AGA AGA CAA TGA AG-3') that anneals to a site located within the cDNA molecule, and a deoxyinosine-containing abridged anchor primer, AAP (5'-GGC CAC GCG TCG ACT AGT ACG GGI IGG GII GGG IIG-3') provided by GIBCO Life Technologies. To increase the quantity of the specific cDNA product the original PCR was re-amplified using the abridged universal amplification primer, AUAP (5'-GGC CAC GCG TCG ACT AGT AC-3', provided by GIBCO Life Technologies) and a second nested gene-specific primer DHRD.91 .s5 (5'-TGC TCC CCA AAA ATA CTC AG-3'). The obtained 200 bp PCR product was directly sequenced using primer DHRD.91 .s5. Upstream of primer DHRD.91 .r12 an additional 45 basepairs of 5' sequences were identified localizing the putative transcriptional start site to position -232 of the cDNA.

Assembly of DHRD.91 .r12/DHRD.91 s11-amplified cDNA and 5'-RACE sequences yielded a 1.990 bp transcript. The cDNA contains an open reading frame (ORF) of 1693 bp with a first potential in-frame translation initiation codon, ATG, starting 73 nucleotides downstream. Therefore, the protein predicted from the ORF consists of 540 amino acid residues, resulting in a calculated molecular mass of 59.4 kDa and an isoelectric point of 9.3.

### Northern blot analysis

Northern blot analysis was performed with total RNA isolated using the guanidinium thiocyanate method (Chomczynski and Sacchi 1987). Each lane contained 12 µg of total RNA from lung, cerebellum, uterus, retina, liver, heart, RPE cell line ARPE-19 (kindly provided by Dr. L.M. Hjelmeland, University of California, Davis), RPE tissue, lymphocytes and was electrophoretically separated in the presence of formaldehyde. Insert of clone ys91a03 was used for filter hybridization at 65°C in 0.5 mM sodium phosphate buffer, pH 7.2; 7% SDS, 1 mM EDTA at 65°C (Church and Gilbert 1984). A 6.5 kb transcript was identified exclusively in cerebellum and retina. To confirm the extend of the 3'-UTR, a 269 bp PCR probe was isolated 2.2 kb downstream of the TAA stop codon of the cDNA by PCR with primers DHRD-3UTR-R (5'-TTA CGC AAA GGA ACA AAG-3') and DHRD-3UTR-F (5'-AAG TGG AAA TGG GCA GAA AG-3') and a 4.5 kb EcoRI subclone containing part of 3'-end of the gene (see below). This probe was hybridized to the same Northern blot filter as specified above and revealed the identical signal of 6.5 kb in size if compared to the hybridization with ys91a03. Together the findings strongly suggests that the pNEU60 gene is intronless within its coding sequence containing 1 intron in the 5'-untranslated region and possessing an extensive 3'-UTR of approximately 4.6 kb.

### Genomic organization

³²P-dCTP-labeled probe ys91a03 was hybridized to high-density membrane filters of a P1-derived artificial chromosone PAC (PAC library) RPCI1 (the library was kindly provided by Dr. P. deJong, Roswell Park Cancer Institute, Buffalo, NY, USA). A total of 6 positive PAC clones were isolated (dJ135O20, dJ187H19, dJ190G15, dJ219G3, dJ302H14, dJ315C2). PAC clone dJ302H14 was digested with restriction enzyme EcoRI, electophoretically separated and blotted onto a nylon membran. Southern blot hybridization was carried out with cDNA probe ys91a03 revealing two hybridization signals of 1.3 and 4.5 kb in size. These fragments were size-selected by elution from agarose gel slices and subsequently subcloned into the EcoRI site of pBluescript KS-II (+) (Stratagene). The 1.3 kb and 4.5 kb inserts were partly sequenced. To determine the exon/intron structure, the genomic sequences were aligned to the complete cDNA using the MacVector software (Kodak). Two exons were identified with exon 1 entirely contained in the 1.3 kb EcoRI fragment and exon 2 entirely contained in the 4.5 kb insert.

### Nucleotide and protein database analyses

To find similar nucleotide sequences in the databases the full length cDNA sequence of pNEU60 was analyzed using the blastx option of the Blast program at NCBI (http://www.ncbi.nlm.nih. gov/cgi-bin/BLAST/nph-blast?Jform=1). No significant matches in the non-redundant DNA sequences were found. Local sequence alignments of the putative protein sequence with known proteins or protein motifs was done using the Blastp and Beauty programs at Baylor College of Medicine (http://dot.imgen.bcm. tmc.edu:9331/seq-search/protein-search.html) and the BIOCCELERATOR at the Weizmann Institute (http://sgbcd.weizmann.ac.il/genweb/index.html). Several motifs were identified including 5 putative N-glycosylation sites at amino acid positions 2-5, 12-15, 25-28, 153-156, 310-313, 420-423 and several putative myristylation sites. To search for the presence of specific motifs within the putative protein sequences the pfscan program at the ProfileScan Server (http://www.isrec.isbsib.ch/profile/databases.html) as well as the TMpred program (http://www.isrec.isbsib.ch/ftp-server/tmpred/www/TMPRED_form.html) were utilized. This identified significant transmembrane (TM) domains and blocks of G protein receptor homologies suggesting a model for a 7 TM topology characteristic of G protein-coupled receptors.

A variety of extracellular signals are transmitted into cells through integral membrane receptors coupled to heterotrimeric GTP-binding proteins (G proteins) which are structurally related to rhodopsin. Examples of this large family of the G protein-coupled receptors include the delta opiate receptor (OPRD1) (Kaufman et al. 1994) or the opioid receptor (OPRM1) (Wang et al. 1994). These receptors are critical for the normal functions of many cell types such as neurons, endocrine cells, cardiac and smooth muscle cells, and sensory cells for detection of light, taste, and smell. Both activating and loss-of-function mutations in G protein-coupled receptors have been found as the cause of human diseases. Diseases associated with loss of G protein-coupled receptor function include nephrogenic diabetes insipidus (V2 vasopressin receptor) (Rosenthal et al. 1992), familial ACTH resistance (ACTH receptor) (Clark et al. 1993), hypergonadotropic ovarian dysgenesis (FSH receptor) (Aittomaki et al. 1995), familial hypothyroidism (TSH receptor) (Heldin et al. 1991) and many others.

### Short description of the Figures

Fig. 1 (SEQ ID NO. 1) shows the nucleotide sequence of the pNEU60 cDNA;

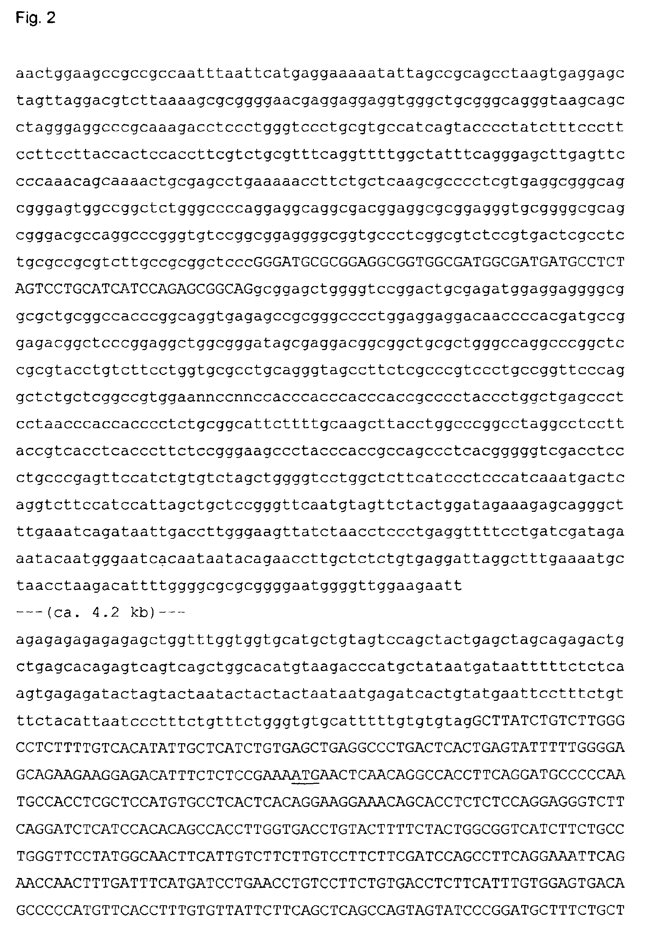

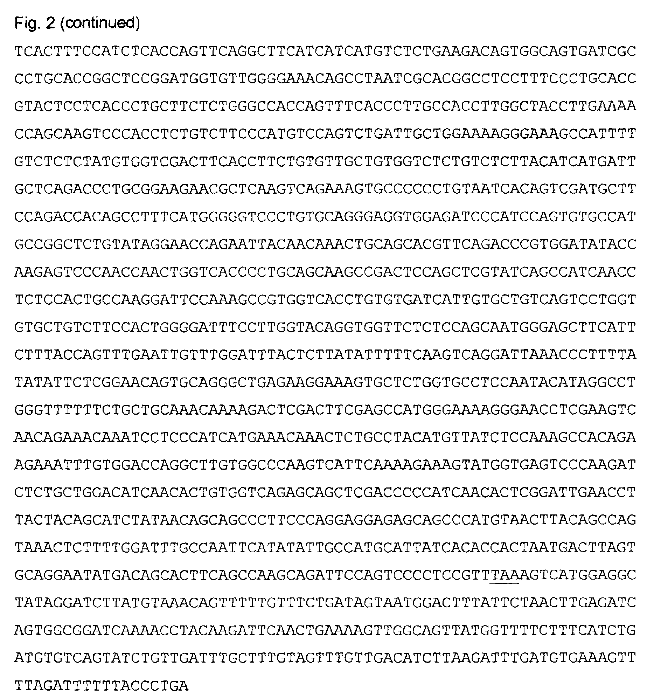

Fig. 2 (SEQ ID NO. 2) shows the nucleotide sequence of the exon/intron organization of the pNEU60 gene;
Fig. 3 (SEQ ID NO.3) shows the amino acid sequence of the predicted pNEU60 protein;

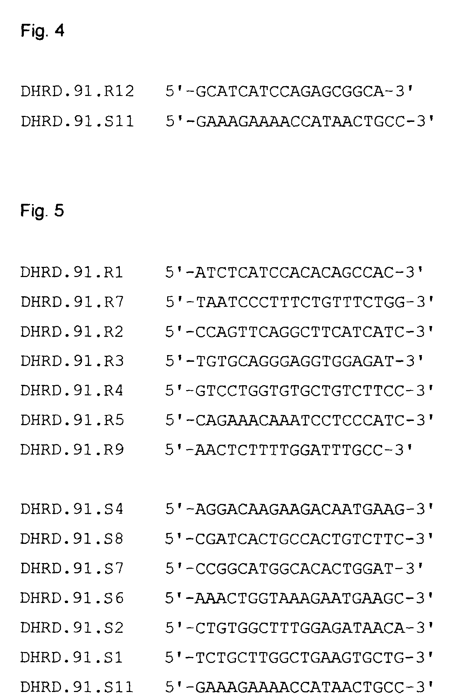

Fig. 4 (SEQ ID NOS. 4 and 5) shows the oligonucleotide PCR primers utilized to amplify the pNEU60 cDNA;
Fig. 5 (SEQ ID NOS. 6-19) shows the oligonucleotide PCR primers that may be used for mutational analyses

### References

Aittomaki K, Lucena JLD, Pakarinen P, Sistonen P, Tapanainen J, Gromoll J, Kaskikari R, Sankila EM, Lehvaslaiho H, Engel AR, Nieschlag E, Huhtaniemi I, de la Chapelle A. Mutation in the follicle-stimulating hormone receptor gene causes hereditary hypergonadotropic ovarian failure. Cell 82: 959-968 (1995).
Allikmets R, Shroyer NF, Singh N, Seddon JM, Lewis RA, Bernstein PS, Peiffer A, Zabriskie NA, Li Y, Hutchinson A, Dean M, Lupski JR, Leppert M. Mutation of the Stargardt disease gene (ABCR) in age-related macular degeneration. Science 277: 1805-1807 (1997).
Banks CN, Hutton WK. Blindness in New South Wales: an estimate of the prevalence and some of the contributing causes. Aust J Ophthalmol 9:285-288 (1981).
Bressler NM, Bressler SB. Preventative ophthalmology. Age-related macular degeneration. Ophthalmology 102:1206-1211(1995).
Bressler NM, Bressler SB, Fine SL. Age-related macular degeneration. Surv Ophthalmol 32:375-413 (1988).
Bressler SB, Bressler NM, Fine SL, Hillis A, Murphy RP, Olk RJ, Patz A. Natural course of choroidal neovascular membranes within the foveal avascular zone in senile macular degeneration. Am J Ophthalmol 93:157-163 (1982).
Chomczynski P, Sacchi N. Single-step method of RNA isolation by acid guanidinium thiocyanate-phenol-chloroform extraction. Anal Biochem 162:156-159(1987).
Church GM, Gilbert W. Genomic sequencing. Proc Natl Acad Sci USA 81:1991-1995 (1984).
Clark AJL, McLoughlin L, Grossman A. Familial glucocorticoid deficiency associated with point mutation in the adrenocorticotropin receptor. Lancet 341: 461-462 (1993).
Donders FC. Beiträge zur pathologischen Anatomie des Auges. Arch Ophthalmol 1: 106-118(1855).
Dryja TP, Briggs CE, Berson EL, Rosenfeld PJ, Abitbol M. ABCR gene and age-related macular degeneration. Science 279:1107 (1998).
Feeney L. Lipofuscin and melanin of human retinal pigment epithelium. Fluorescence, enzyme cytochemical, and ultrastructural studies. Invest Ophthalmol Vis Sci 17:583-600 (1978).
Feeney-Burns L, Ellersieck MR. Age-related changes in the ultrastructure of Bruch's membrane. Am J Ophthalmol 100:686-697(1985).
Frohman MA, Dush MK, Martin GR. Rapid production of full-length cDNAs from rare transcripts: amplification using a single gene-specific oligonucleotide primer. Proc Natl Acad Sci USA 85:8998-9002 (1988).
Gass JD. Drusen and disciform macular detachment and degeneration. Arch Ophthalmol 90:206-217 (1973).
Gass JD. Pathogenesis of disciform detachment of the neuroepithelium. Am J Ophthalmol 63: Suppl:1-139(1967).
Ghafour IM, Allan D, Foulds WS. Common causes of blindness and visual handicap in the west of Scotland. Br J Ophthalmol 67: 209-213 (1983).
Green WR, McDonnell PJ, Yeo JH. Pathologic features of senile macular degeneration. Ophthalmology 92:615-27 (1985).
Grey RH, Burns-Cox CJ, Hughes A. Blind and partial sight registration in Avon. Br J Ophthalmol 73:88-94(1989).
Heiba IM, Elston RC, Klein BE, Klein R. Sibling correlations and segregation analysis of age-related maculopathy: the Beaver Dam Eye Study. Genet Epidemiol 11:51-67 (1994).
Heldin NE, Gustavsson B, Westermark K, Westermark B. A somatic point mutation in a putative ligand binding domain of the TSH receptor in a patient with autoimmune hyperthyroidism. J Clin Endocr Metab 73: 1374-1376 (1991).
Hogan MJ, Alvarado J. Studies on the human macula. IV. Aging changes in Bruch's membrane. Arch Ophthalmol 77:410-420(1967).
Holz FG, Sheraidah G, Pauleikhoff D, Bird AC. Analysis of lipid deposits extracted from human macular and peripheral Bruch's membrane. Arch Ophthalmol 112:402-406 (1994).
Hyman LG, Lilienfeld AM, Ferris FL, Fine SL. Senile macular degeneration: a case-control study. Am J Epidemiol 118:213-227 (1983).
Hyman L. Epidemiology of eye disease in the elderly. Eye 1: 330-341 (1987).
Kaufman DL, Xia YR, Keith DE, Newman D, Evans CJ, Lusis AJ. Localization of the delta-opioid receptor gene to mouse chromosome 4 by linkage analysis. Genomics 19: 405-406(1994).
Klaver CCW, Assink JM, Bergen AAB, vanDuijn CM. ABCR gene and age-related macular degeneration. Science 279:1107 (1998).
Klein R, Klein BE, Franke T. The relationship of cardiovascular disease and its risk factors to age-related maculopathy. The Beaver Dam Eye Study. Ophthalmology 100:406-414 (1993).
Leibowitz HM, Krueger DE, Maunder LR, Milton RC, Kini MM, Kahn HA, Nickerson RJ, Pool J, Colton TL, Ganley JP, Loewenstein JI, Dawber TR. The Framingham Eye Study monograph: An ophthalmological and epidemiological study of cataract, glaucoma, diabetic retinopathy, macular degeneration, and visual acuity in a general population of 2631 adults, 1973-1975. Surv Ophthalmol 24(Suppl): 335-610 (1980).
Maguire P, Vine AK. Geographic atrophy of the retinal pigment epithelium. Am J Ophthalmol 102:621-625 (1986).
Meyers SM, Zachary AA. Monozygotic twins with age-related macular degeneration. Arch Ophthalmol 106:651-653 (1988).
Paetkau ME, Boyd TA, Grace M, Bach-Mills J, Winship B. Senile disciform macular degeneration and smoking. Can J Ophthalmol 13:67-71 (1978).
Pauleikhoff D, Harper CA, Marshall J, Bird AC. Aging changes in Bruch's membrane. A histochemical and morphologic study. Ophthalmology 97:171-178(1990).
Pauleikhoff D, Wormald RP, Wright L, Wessing A, Bird AC. Macular disease in an elderly population. Ger J Ophthalmol 1:12-15 (1992).
Piguet B, Wells JA, Palmvang IB, Wormald R, Chisholm IH, Bird AC. Age-related Bruch's membrane change: a clinical study of the relative role of heredity and environment. Br J Ophthalmol 77:400-403 (1993).
Pizzarello LD. The dimensions of the problem of eye disease among the elderly. Ophthalmology 94:1191-5 (1987).
Rosenthal W, Seibold A, Antaramian A, Lonergan M, Arthus MF, Hendy GN, Birnbaumer M, Bichet DG. Molecular identification of the gene responsible for congenital nephrogenic diabetes insipidus. Nature 359: 233-235 (1992).
Sarks SH. Ageing and degeneration in the macular region: a clinico-pathological study. Br J Ophthalmol 60:324-341 (1976).
Seddon JM, Ajani UA, Mitchel BD. Familial aggregation of age-related maculopathy. Am J Ophthalmol 123: 199-206 (1997).
Silvestri G, Johnston PB, Hughes AE. Is genetic predisposition an important risk factor in age-related macular degeneration? Eye 8:564-568 (1994).
Sperduto RD, Hiller R. Systemic hypertension and age-related maculopathy in the Framingham Study. Arch Ophthalmol 104:216-219 (1986).
The Eye Disease Case-Control Study Group. Risk factors for neovascular age-related macular. Arch Ophthalmol 110:1701-1708 (1992).
Wang JB, Johnson PS, Persico AM, Hawkins AL, Griffin CA, Uhl GR. Human mu opiate receptor: cDNA and genomic clones, pharmacologic characterization and chromosomal assignment. FEBS Lett 338:217-222(1994).
Yap M, Weatherill J. Causes of blindness and partial sight in the Bradford Metropolitan District from 1980 to 1985. Ophthalmic Physiol Opt 9:289-292 (1989).
Young RW. Pathophysiology of age-related macular degeneration. Surv Ophthalmol 31:291-306 (1987).

## Claims

1. A protein which comprises the same or substantially the same amino acid sequence as that represented by SEQ ID NO. 3, or a variant of the amino acid sequence having a deletion, addition or substitution of 1 to 10 amino acids, or its salt.

2. The protein as claimed in claim 1 which comprises the same or substantially the same amino acid sequence as that represented by SEQ ID NO. 3, or its salt.

3. A partial peptide of the protein as claimed in claim 1 or 2 having at least 200 amino acids, or its salt.

4. An isolated cDNA comprising
a) DNA having a nucleotide sequence encoding the protein as claimed in any of claims 1 to 4,
b) a DNA hybridizing with the DNA of a), or
c) a DNA which is synonymous to a) or b) due to the degeneracy of the genetic code.

5. The isolated DNA sequence according to claim 4 having
a) the nucleotide sequence represented by SEQ ID NO. 1,
b) DNA hybridizing with the DNA of a), or
c) a DNA which is synonymous to a) or b) due to the degeneracy of the genetic code.

6. A DNA comprising a nucleotide sequence with at least a 65 % homology with the nucleotide sequence according to claims 4 or 5.

7. A recombinant vector comprising the DNA according to any of claims 4 to 6.

8. A transformant comprising the recombinant vector according to claim 7.

9. A process for preparing the protein according to claims 1 or 2 which comprises cultivating the transformant according to claim 8 to form the protein according to claims 1 or 2 or its salt and recovering it.

10. A method of screening for modulators and interacting proteins or factors comprising the natural cellular interacting substances of pNEU60.

11. A method as claimed in claim 10 for screening chemical libraries.

12. A kit for screening for compounds which interacts with at least one DNA or protein domain which comprises an essential element of the DNA or protein function according to any of claims 1 to 3 or its salt.

13. A compound which alters / reacts with at least one epitope of the protein according to any of claims 1 to 3 or its salts obtained by the screening method according to claims 10 or 11.

14. An antibody against the protein according to any of claims 1 to 3 or its salt.

15. A method of determining the protein according to any of claims 1 to 3 or its salts which comprises bringing the antibody according to claim 14 into contact with the protein according to any of claims 1 to 3 or its salt in a specimen.

16. A pharmaceutical composition comprising as an effective component the protein according to any of claims 1 to 3 or its salt, and a pharmaceutically acceptable carrier or diluent.

17. A pharmaceutical composition comprising as an effective component the compound according to claim 13 or its salt, and a pharmaceutically acceptable carrier or diluent.

18. Generation of animal models targeting the pNEU60 gene or pad of it, which targeting methods include knock-in, knock-out and transgenic constructs derived from cDNA and genomic sequences of the pNEU60 gene.
